Europäisches Patentamt

European Patent Office

Office européen des brevets

(11) Publication number: **0 446 350 A1**

(12) **EUROPEAN PATENT APPLICATION**
published in accordance with Art. 158(3) EPC

(21) Application number: 89900894.0

(22) Date of filing: 23.12.88

(86) International application number:
PCT/JP88/01311

(87) International publication number:
WO 89/05851 (29.06.89 89/14)

(51) Int. Cl.⁵: **C12N 9/16**

(30) Priority: 24.12.87 JP 325255/87

(43) Date of publication of application:
18.09.91 Bulletin 91/38

(84) Designated Contracting States:
**DE FR GB**

(71) Applicant: **TEIJIN LIMITED**
**11, Minamihonmachi 1-chome, Higaski-ku**
**Osaka-shi, Osaka 541(JP)**

(72) Inventor: **IMAIZUMI, Atsushi**
**10-8, Nishihirayama 5-chome**
**Hino-shi Tokyo 191(JP)**
Inventor: **SUWA, Yorimasa**
**403, Shanzeru Takao 1262, Hatsuzawa-cho**
**Hachioji-shi, Tokyo 193(JP)**
Inventor: **OKADA, Masahiro**
**5-18, Tamadaira 3-chome**
**Hino-shi Tokyo 191(JP)**
Inventor: **SUZUKI, Yoji**
**20-2, Tamadaira 5-chome**

**Hino-shi Tokyo 191(JP)**
Inventor: **KUDO, Ichiro**
**28-15, Minamisenzoku 1-chome**
**Ota-ku Tokyo 145(JP)**
Inventor: **INOUE, Keizo**
**3-17-605, Etchujima 1-chome**
**Koto-ku Tokyo 135(JP)**
Inventor: **HARA, Shuntaro**
**4-43, Honmachi 2-chome**
**Shiki-shi Saitama 353(JP)**
Inventor: **MATSUTA, Kunio**
**27-24-1103, Yato-cho 3-chome**
**Tanashi-shi Tokyo 188(JP)**
Inventor: **MIYAMOTO, Terumasa**
**43-4, Ogikubo 3-chome**
**Suginami-ku Tokyo 167(JP)**

(74) Representative: **Votier, Sidney David et al**
**CARPMAELS & RANSFORD 43, Bloomsbury**
**Square**
**London WC1A 2RA(GB)**

(54) **NOVEL HUMAN PHOSPHOLIPASE A2 AND ITS FRAGMENT PEPTIDE.**

(57) A protein comprising phospholipase A₂ which has an N-terminal amino acid sequence of NH₂-Asn-Leu-Val-Asn-Phe-X-X-Met-Ile-X-Leu-Thr-Thr-Gly-Lys-Glu-Ala-Ala-Leu-X-Tyr-Gly-Phe-Tyr-Gly-, for example, a protein having a molecular weight of about 137,000 and originating in the human inflamed region, and a polypeptide or a fragment thereof having the above-described amino acid sequence participate in inflammation and act as an inflammation inducer, thus being useful as antigens for diagnosis of diseases such as inflammation, infection, immunological diseases, etc.

EP 0 446 350 A1

Technical Field

The present invention relates to a protein having phospholipase A₂ activity with a specific amino acid sequences on its N-terminal.

Background of the Art

Phospholipase A₂ is an enzyme to hydrolyze the B-ester bond of phosphatidylcholine into lysophosphatidylcholine and a fatty acid, and has been known to occur in mammalian pancreas, snake venom, bee venom or the like. The primary structure has been already determined on about 35 proteins originating from anake venoms, about 5 from mammalian pancreas and one from bee venom among the proteins having phospholipase A₂ activity [Seikagaku (Japan) 57 (1), 47 - 54]. These encymes whose primary structures have been determined are classified into the secretory phospholipase A₂ and a variety of studies at the molecular level have been done using them, since their separation and purification are simple, due to their relatively large occurrence. They tole us that the phospholipase A₂s which have been known up to now have relatively low molecular weight (13,000 - 14,000) and show considerably high stability even at elevated temperature, in extremely acidic or alkaline solutions, or in solutions containing a high concentration of a denaturing agent (for example, urea, guanidine hydrochloride, a variety of organic solvent), as they contain many S-S bonds (6 - 7 bonds). Many phospholipase A₂s are acidic or neutral proteins, but some of them are strongly basic and regarded as the main body of snake venom and bee venom, since they exhibit strong physiological actions such as neurotoxic, musculotoxic or cardiotoxic actions.

It has been known that mammalian pancreatic phospholipase A₂ are secreted in the form of a proenzyme, and undergo restricted hydrolysis into the active form of phospholipase A₂ by losing the heptapeptide fragment at the N-terminal. X-ray crystal structure analysis have been reported in considerable detail on phospholipase A₂s originating from bovine and porcine pancreas. The phospholipase A₂ originating from human pancreas was confirmed on its primary amino acid sequence (Vathoij.H.M. et al 1983 Biochim. Biophys. Acta 74. 793-99), but the X-ray crystallographic studies have not yet been carried out. The human pancreatic phospholipase A₂ was cloned recently and its amino acid sequence was elucidated at the gene level (Seilhemer.J.J. Randall.T.L. Yamanaka.M. and Johonson L.K. 1986 DNA. Vol. 15 519-527). These pancreatic phospholipase A₂s are thought to act as a digestive enzyme which is secreted from pancreas.

In the meantime, phospholipase A₂ activity has been observed over a wide range of organ tissues and cells. These facts suggest the existence of other phospholipase A₂ than mammalian pancreatic phospholipase A₂. For example, brain, liver, lungs, spleen, small intestine, white blood cells, red blood cells, platelets, macrophage, cartilage cells, inflamed tissues showed phospholipase A₂ activity, and the isolation and purification of these phospholipase A₂s were tried.

Among these enzymes, human phospholipase A₂ has been known to be an important enzyme which suppresses the usual phospholipid metabolism and membrane fluid flux as well as plays important roles in the initial step of arachidonic acid metabolism. Therefore, the elucidation of the structure and functions of these enzymes has presumably great significance in order to control the diseases concerning inflammation, infections and immunity. Up to now, however, human phospholipase A₂s other than pancreatic one have not yet been clarified on the complete amino acid sequence, although the isolation and purification were partially tried. Only a part of the amino acid sequence turned out in the phospholipase A₂ originating from porcine small intestine. (Varger.R.F. et al 1982 Biochemistry 21 3883 - 3889). It is thought to be due to smaller occurrence of these phospholipase A₂s compared with the snake venom phospholipase A₂ and human pancreatic phospholipase A₂.

Recently, Inoue et al isolated and purified the phospholipase A₂ formed in rat peritoneal cavity, when casein is given to the rat, and examined its phoperties [Seikagaku (Japan) 58 (8) 766 1986]. According to their report, subcutaneous injection of 100 ng of the purified enzyme on rat backs increased vascular permeability to cause transudation of Evans Blue which was intravenously given beforehand. Meanwhile, the mammalian pancreatic phospholipase A₂ has been confirmed to have not this action. Thus, the existence of other phospholipase A₂s clearly different from known ones in their roles in vivo is suggested, and has received attention as an inflammation-specific phospholipase A₂.

As for such human phospholipase A₂ presumably participating inflammation, increased activity of phospholipase A₂ has been observed in a variety of diseases such as psoriatic skins, septic serum, synovial fluid in patients with rheumaoid arthritis, and the relationship between these phospholipase A₂s and inflammations has aroused interest.

As aforesaid, the elucidation of the structure and functions of such human phospholipase $A_2$ has great significance from the standpoint of the therapy of diseases relating to inflammation, infection and immunity. Nonetheless, the research hardly has been done on these problems, because such phospholipase $A_2$ is almost unavailable, because they are difficult to be isolated and purified.

Disclosure of the Invention

In consideration of such problems in the prior arts, the present inventors have made intense study on the phospholipase $A_2$ which has an amino acid primary sequence different from that in the pancreatic phospholipase $A_2$, in particular, the phospholipase $A_2$ which would be concerned with inflammation and would have proinflammatory effect, and reached the present invention.

Namely, the present invention is
the protein which has the amino acid sequence at the N-terminal of the following formula [I]:

$$NH_2\text{-Asn-Leu-Val-Asn-Phe-X-X-Met-Ile-X-Leu-Thr-Thr-Gly-}$$
$$\text{Lys-Glu-Ala-Ala-Leu-X-Tyr-Gly-Phe-Tyr-Gly-} \qquad [I]$$

and has the phospholipase $A_2$ activity, and
the protein which has the amino acid sequence at the N-terminal of following formula [II]:

$$NH_2\text{-Asn-Leu-Val-Asn-Phe-His-Arg-Met-Ile-Lys-Leu-Thr-}$$
$$\text{Thr-Gly-Lys-Glu-Ala-Ala-Leu-Ser-Tyr-Gly-Phe-Tyr-Gly-Cys-X-}$$
$$\text{Cys-Gly-Val-Gly-Gly-Arg-Gly-} \qquad [II]$$

and has the phospholipase $A_2$ activity.

In particular, the present invention is the protein which originates from human inflamed sites, has a molecular weight of about 13,700 with the aforesaid amino acid sequence at its N-terminal, and shows the phospholipase $A_2$ activity.

Moreover, the present invention is the peptides which have at least the amino acid sequences of the formulas [I] and [II], and the polypeptides which have the amino acid sequences of the formulas [I] and [II] and their fragments. X in formulas [I] and [II] represents an amino acid residue unidentified with the sequencer, although its existence was confirmed.

Brief Description of Drawings

Fig. 1 shows the elution pattern of the subject protein in affinity chromatography with heparin Sepharose CL6B.

Fig. 2 discloses the elution pattern of the subject protein in the hydrophobic gel column chromatography.

Fig. 3 gives the elution pattern of the subject protein in reversed phase high performance liquid chromatography (HPLC).

Fig. 4 shows the SDS-PAGE of the subject protein purified by reversed phase HPLC.

Fig. 5 gives the calcium ion dependency of the subject protein for the phospholipase $A_2$ activity.

Fig. 6 gives the pH dependency of the subject protein for the phospholipase $A_2$ activity.

Fig. 7 gives the kinetic data showing the substrate specificity of the subject protein.

Fig. 8 shows the activity of the phospholipase $A_2$ in synovial fluid in patients with rheumatoid arthritis and osteoarthritis.

The Best Embodiment of the Invention

Here the substrate specificity of the protein having the phospholipase $A_2$ activity according to the present invention will be discussed. The studies on pancreatic or snake venom phospholipase $A_2$ told us

EP 0 446 350 A1

that a variety of phospholipase $A_2$s have different substrate specificity from one another depending on their origins, and the enzymatic activities also are influenced by their dispersion state. In general, phospholipase $A_2$ shows low activity to a monomeric substrate, but it manifests markedly high activity to a micellar substrate. It is presumed that the phospholipase $A_2$ molecule has the site to recognize the interface between the water phase and the substrate micelle in addition to the active site to effect the catalytic reaction. Mammalian pancreatic phospholipase $A_2$ is a proenzyme, both of the proenzyme and the active form enzyme have almost the same level of affinity to essential metal ion $Ca^{++}$, and shows the same grade of phospholipase $A_2$ activity to the monomeric substrate. The difference between the proenzyme and the active form is in the activity to the micellar substrate, namely the former reveals only low activity, while the latter, much higher activity. This suggests that the restrictive hydrolysis of the proenzyme forms the interface recognition site near the N-terminal. Thus, the amino acid sequence at the N-terminal is the important part for substrate recognition in vivo. In other words, the protein according to the present invention includes a novel type of N-terminal amino acid sequence closely relating to the substrate specificity as well as the phospholipase $A_2$ activity.

The isolation and purification of the protein having phospholipase $A_2$ activity according to the present invention will illustrated here.

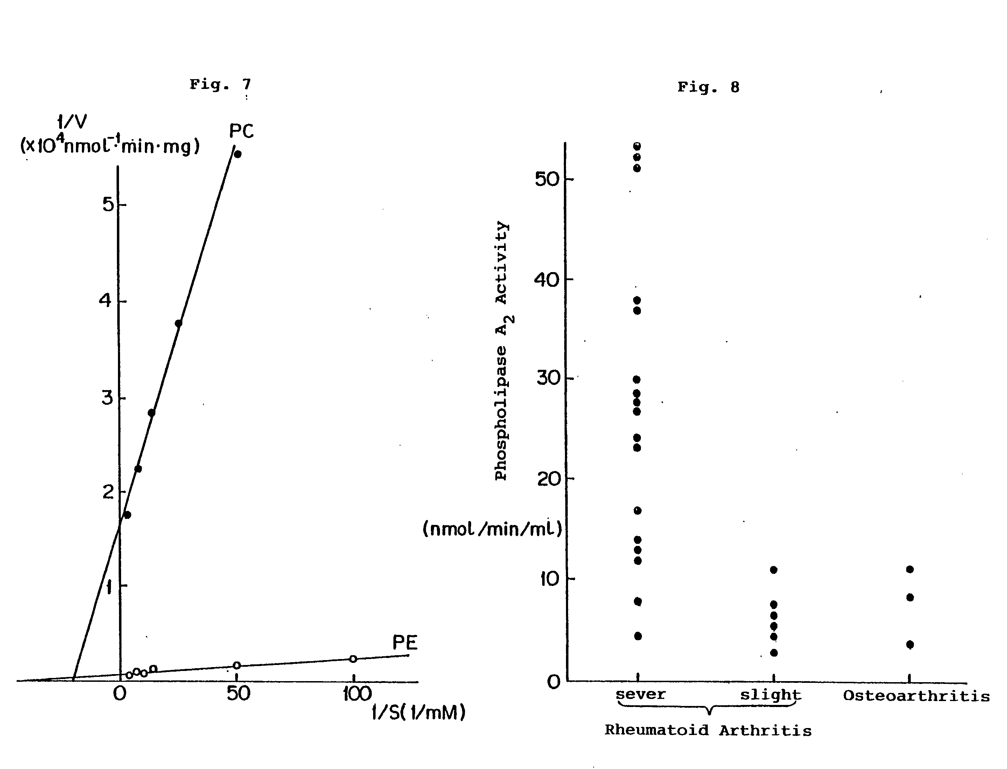

As a starting substance for isolation and purification of phospholipase $A_2$ according to the present invention, is preferably used the fluid from inflamed sites, particularly the synovial fluid from the patients with rheumatoid arthritis. The present inventors focused their attention on rheumatoil arthritis and osteoarthritis as inflammatory diseases and determined the phospholipase $A_2$ activity of the synovial fluid. As shown in Fig. 8, the increase in phospholipase $A_2$ activity was noticed in the patients with rheumatoid arthritis, especially in the patients of high severity, while low activity was observed in osteoarthritis.

Phospholipase $A_2$ according to the present invention is isolated and purified, for example, using synovial fluid collected from the patients with rheumatoid arthritis, as a starting substance, as follows: The starting substance is applied to, for example, heparin Sepharose affinity chromatography, and the phospholipase $A_2$ activity is determined on individual fractions to obtain an activity peak. Then, the peak is subjected to hydrophobic gel chromatography and reversed phase chromatography or the like to purify the subject protein. The final preparation migrates as a single band on SDS-PAGE. This method enables the isolation of about 10 μg of the protein with phospholipase $A_2$ activity from about 400 ml of the fluid. The protein has a molecular weight of about 13,700, requires $Ca^{++}$ ion with the optimal calcium ion concentration near 4 mM, hydrolyzes phosphatidylethnolamine in preference to phosphatidylcholine at the optimal pH of about 9.5, and has the amino acid sequence from the N-terminal of $NH_2$-Asn-Leu-Val-Asn-Phe-X-X-Met-Ile-X-Leu-Thr-Thr-Gly-Lys-Glu-Ala-Ala-Leu-X-Tyr-Gly-phe-Tyr-Gly-, in more detail, $NH_2$-Asn-Leu-Val-Asn-Phe-His-Arg-Met-Ile-Lys-Leu-Thr-Thr-Gly-Lys-Glu-Ala-Ala-Leu-Ser-Tyr-Gly-Phe-Tyr-Gly-Cys-X-Cys-Gly-Val-Gly-Gly-Arg-Gly. The comparison of these properties with those of human pancreatic phospholipase $A_2$ which is so far known has told us that the protein is a new type of phospholipase $A_2$ of a quite novel structure. Here, the protein with phospholipase $A_2$ activity according to the present invention has been explained on its isolation and purification from synovial fluid in patients with rheumatoid arthritis, but the present invention is not limited to these origin and process. The protein may be isolated and purified from animals other than human, or from microorganisms and mammalian cells through recombinant DNA techniques. Any proteins are included in the present invention, as long as they have the N-terminal amino acid sequence defined as stated above in addition to the phospholipase $A_2$ activity.

Since phospholipase $A_2$ according to the present invention reveals markedly high activity in in inflamed sites, in particular in synovial fluid in patients with rheumatoid arthritis, the measurement of the amont and activity of the enzyme gives a possibility of diagnosis or therapy of rheumatoid arthritis. In such cases, the protein and fragment can be useful as antigens for the immunoassay. Further, a therapy for cancer in which an inflammation is caused on the tumor to expel the tumor cells has been recently reported, and it is not only a dream to heal cancers, when the proinflammatory effect of the protein is utilized to cause the inflammation. The participation of phospholipase $A_2$ to repair of cell membranes is suggested, and the possibility of use as a vulnerary is also pointed out. Further, an inhibitor specific to the enzyme has a possibility of use as an anti-inflammatory, and the enzyme is one of essential protein as an enzyme source for screening such inhibitors. Moreover, the polypeptides according to the present invention, having the amino acid sequence of formulas [I] and [II], and their fragments have a possibility of inhibiting the phospholipase $A_2$ activity in the competition with the substrate, thus a possibility of use as an inhibitor is also suggested.

Among the formulas [I] and [II], the amino acid sequence of 2 to 25 or 2 to 30 in an arbitrary part are cited as such fragments, and in particular, the sequence the 5th to 7th from the N-terminal is expected to have inhibitory action on phospholipase $A_2$ in relation to the substrate specificity as stated above.

4

In these days when the roles of phospholipase $A_2$ in vivo is being made clear, the utility value of the proteins and polypeptides provided by the present inventors is great.

The tests and measurements which have been utilized in the present invention are as follows:

(1) Determination of phospholipase $A_2$ activity:

$^{14}C$ acetic acid was incorporated into E. coli and phosphatidylethanolamine labeled with $^{14}C$ was extracted therefrom for use as a substrate. Samples were added into the reaction system containing 100 mM Tris-HCl (pH 9.0), 4 mM $CaCl_2$, 50 nmol $^{14}C$-phosphatidylethanolamine and incubated at $37°C$ for 30 minutes. Then, the reactions were stopped with the Dole's reagent. The labeled fatty acid which had been formed by hydrolysis of the phosphatidylethanolamine was extracted with heptane, and determined with a liquid scintillation counter.

(2) SDS-polyacrylamide gel electrophoresis (SDS-PAGE)

A part of the samples which had been fractionated by a variety of chromatographies was heated together with 1 % SDS and 2-mercaptoethanol at $100°C$ for 10 minutes. Then, the sample was applied to 12.5 % polyacrylamide gel, then subjected to electrophoresis at an electric current of 15 mA for 1.5 hours. After completion of electrophoresis, the gel was stained with the silver stain kit of Bio-Rad Co.

(3) Primary structure of proteins:

The amino acid sequence was determined with a gas-phase protein sequencer (Applied Biosystem, model 477A). In other words, about 2 $\mu$g of purified samples was dissolved in 30 $\mu$l of trifluoroacetic acid (TFA) and applied to the gas-phase protein sequencer. The sample automatically underwent Edman degradation and the phenylhydantoin (PTH)-amino acid derivatives formed were identified with HPLC (Applied Biosystem, Model-120A) as individual amino acids.

〈Examples〉

The present invention will be illustrated in more detail by following examples:

Example 1

Purification and isolation of proteins with phospholipase pase $A_2$ activity

(a) Isolation and purification of the subject protein with heparin-Sepharose chromatography

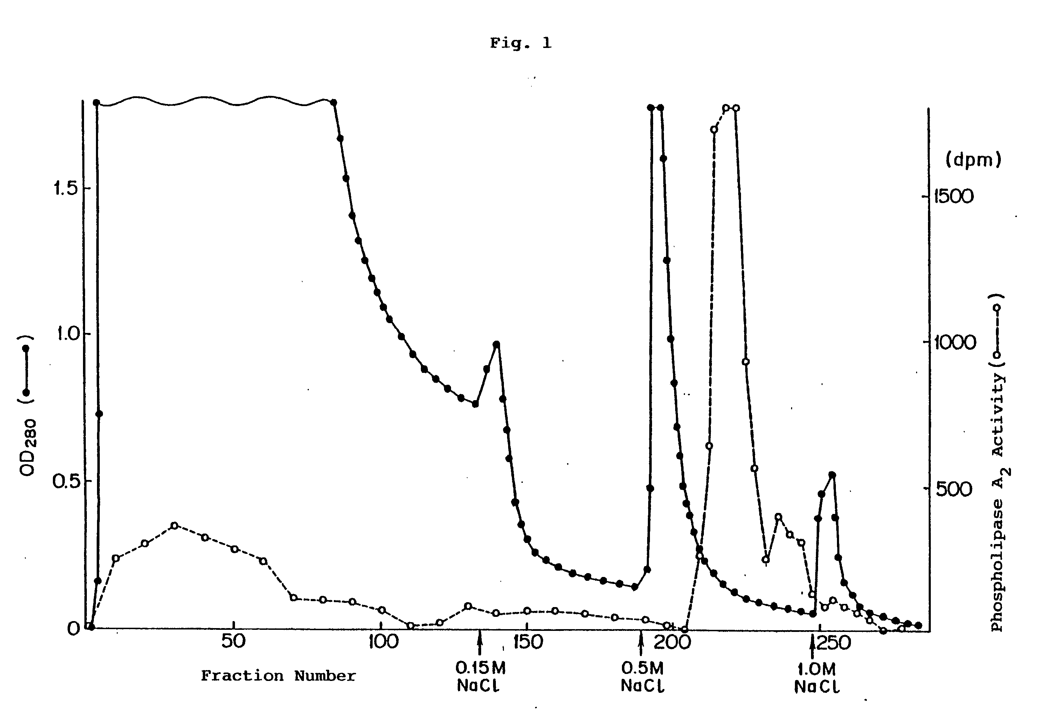

About 420 ml of synovial fluid from patients with rheumatoid arthritis were centrifuged to remove cells and other solid components, and the supernatant was applied to a heparin-Sepharose CL6B column (25 mm diameter x 80 mm) equilibrated with 20 mM Tris-HCl buffer and eluted with 0.15 - 1.0 M NaCl at a flow rate of 0.3 ml/min. The result is given in Fig. 1. In the figure, the dashed line shows the activity of phospholipase $A_2$, while the solid line gives the amount of the protein monitered at UV 280 nm. Marked phospholipase $A_2$ activity was eluted with about 0.7 M NaCl concentration gradient.

(b) Isolation and purification of the subject protein by hydrophobic gel column chromatography

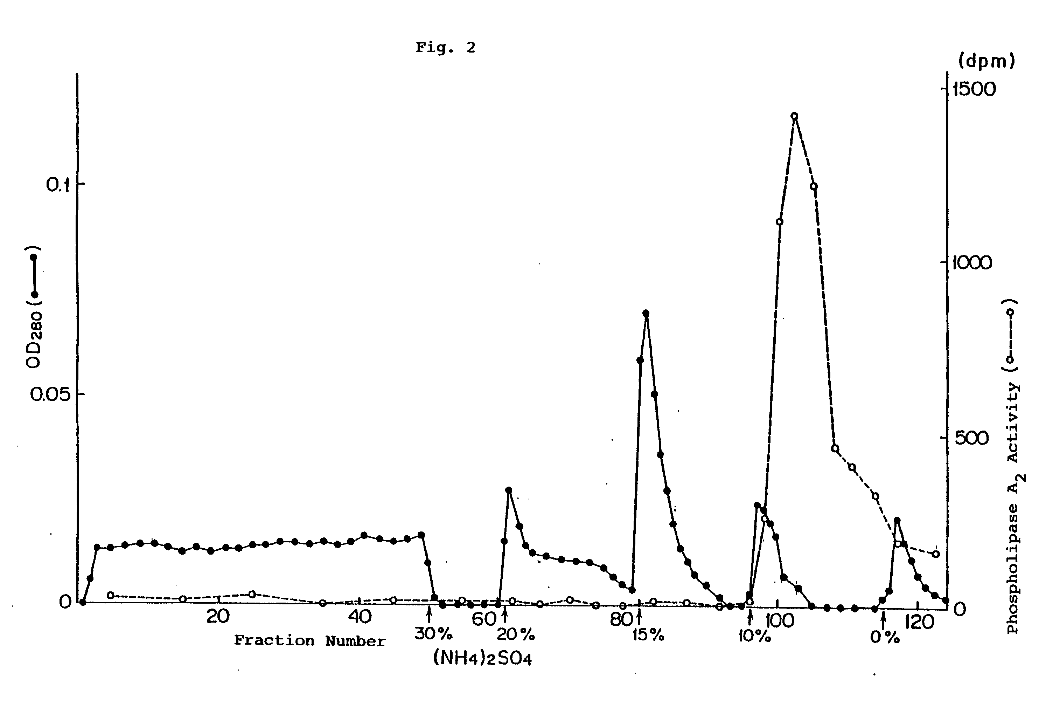

For more purification of the above-stated fraction having phospholipase $A_2$ activity, the fractions were pooled and applied to hydrophobic column chromatography (Butyltoyopearl$^R$ 650 column, 10 mm diameter x 275 mm) at a flow rate of 0.7 ml/min. Elution was conducted using solutions containing 20 mM Tris-HCl of pH 7.4, 1 M NaCl and ammonium sulfate of concentrations changing stepwise 30 %, 20 %, 15 %, 0 %. The phospholipase $A_2$ activity of individual elutes are shown in Fig. 2. The dashed line represents the phospholipase $A_2$ activity, while the solid line displays the protein amount monitered at UV 280 nm. The phospholipase $A_2$ activity was noticed in 10 % ammonium sulfate. The SDS-PAGE of this active fractions revealed the necessity of further purification.

(c) Isolation and purification of the subject protein by reversed phase high performance liquid chromatography (HPLC)

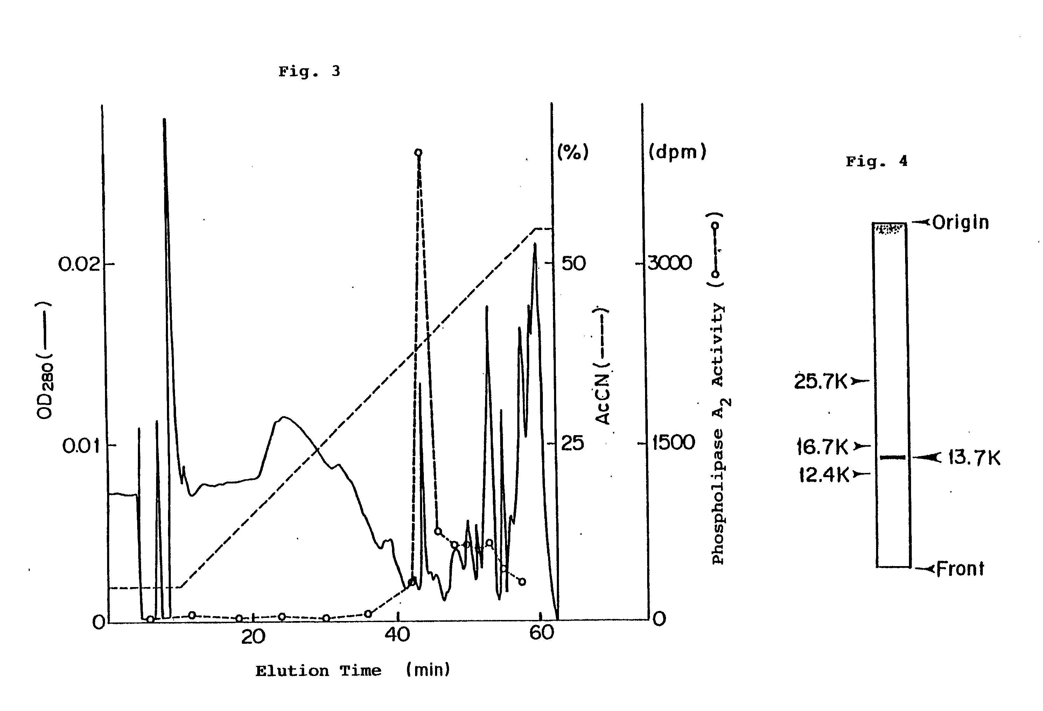

The phospholipase $A_2$-active fractions in the hydrophobic gel column chromatography was applied to reversed phase HPLC column (TSK-gel ODS-120T, 4.6 mm diameter x 150 mm) equilibrated with 0.1 % TFA. The subject protein was eluted with acetonitril of linear concentration gradient shown with the dashed line in Fig. 3. The phospholipase $A_2$ activity was detected in the fractions marked with the bold dashed line and open circles in Fig. 3. SDS-PAGE of the fraction is given in Fig. 4. The subject phospholipase $A_2$ was a single bnad of about 13,700 molecular weight.

Example 2

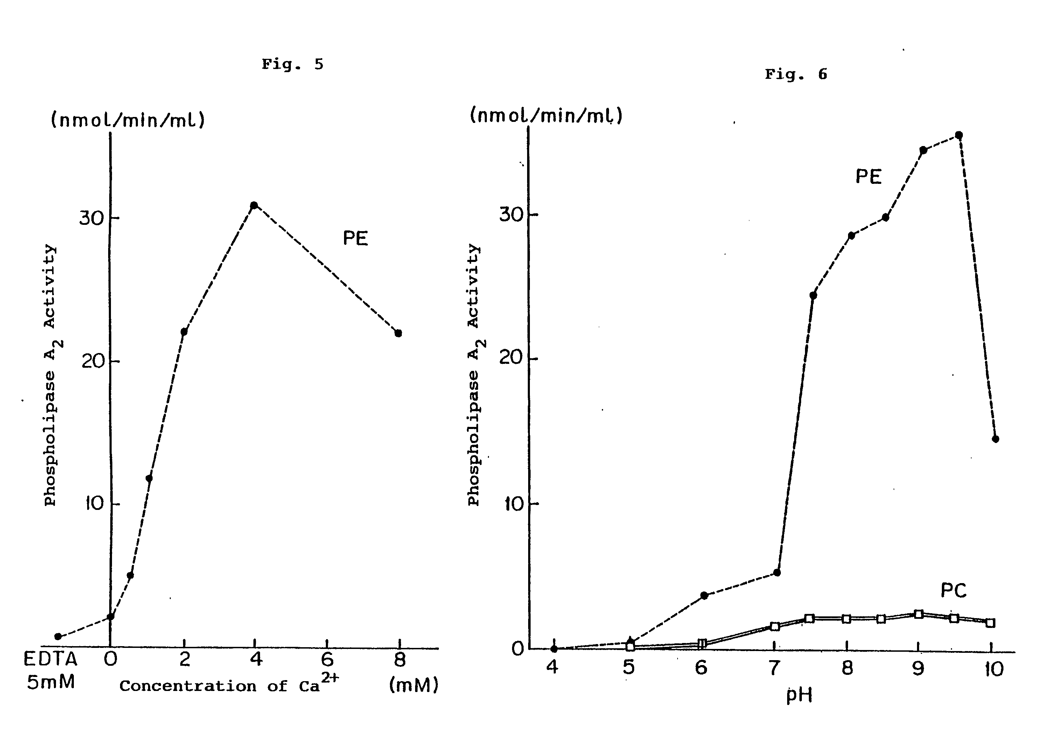

In order to clarify biochemical properties of the protein with phospholipase $A_2$ activity which was isolated and purified in Example 1, the calcium ion requirement, optimal pH and substrate specificity were investigated by measuring the phospholipase $A_2$ activity as the calcium ion concentration was changed, the pH of the Tris-HCl buffer was altered, and phosphatidylethanolamine (PE) and phosphatidylcholine (PC) was used as substrates. The results are given in Fig. 5 through 7. In Fig. 5, the phospholipase $A_2$ activity with various concentration of $Ca^+$ is shown, when PE was used as a substrate. As evidently shown in Fig. 5, calcium requirement was seen in the protein according to the present invention, as in phospholipase $A_2$s which were previously characterized, and the optimal calcium ion concentration was about 4 mM. In Fig. 6, the dashed line and the double line represent the optimal pHs of the protein, when PE and PC were used as substrates, respectively. The figure revealed that the protein according to the present invention has its optimal pH near 9 and hydrolyzes PE in preference to PC. Fig. 7 gives the kinetic data showing the substrate specificity of the protein, and the protein is evidently more specific to PE which is shown with open circle-lines than to PC shown with the dense circle-lines.

Example 3

A part of the primary structure of the protein in Example 1 was determined by the gas-phase protein sequencer. The amino acid sequence of the phospholipase $A_2$ at the N-terminal was found to be
$NH_2$-Asn-Leu-Val-Asn-Phe-X-X-Met-Ile-X-Leu-Thr-Thr-Gly-Lys-Glu-Ala-Ala-Leu-X-Tyr-Gly-Phe-Tyr-Gly-
where X represents the residue of the amino acid which could not be identified.

Example 4

The sample which was isolated and purified in Example 1 was used to determine the amino acid composition and a part of the primary structure.
(a) Amino acid composition:
The sample was hydrolyzed with hydrochloric acid, reduced with dithiothreitol in 6 M guanidine-HCl/2 mM EDTA/0.5M Tris-HCl (pH 8.1) at 37°C for 2 hours to cleave the disulfide bonds, then carboxymethylamidated with iodoacetamide. Then, the amino acid composition of the sample (0.5 μg) was determined by the o-phthalaldehyde post-column derivatization method according to Ishida et al (J. Chromatogra. 204, 143-148, 1981).
The result is given in Table 1.

Table 1

| Asx | 7.1 |
|-----|-----|
| Glx | 6.6 |
| Ser | 6.1 |
| Gly | 9.9 |
| His | 2.3 |
| Arg | 8.6 |
| Thr | 6.8 |
| Ala | 7.3 |
| Pro | 2.9 |
| Tyr | 6.3 |
| Val | 2.6 |
| Met | 0.8 |
| Ile | 2.8 |
| Leu | 4.9 |
| Phe | 3.7 |
| Lys | 7.3 |
| Cys | 14.0[d] |
| Trp | n.d.[e] |

Note
d: S-carboxymethylcysteine
e: unidentified

(b) Primary structure of the protein
The primary structure was determined by the gas-phase protein sequencer, as in Example 3.

Resultantly, the amino acid sequence of this phospholipase $A_2$ at the N-terminal was as follows:

```
Asn-Leu-Val-Asn-Phe-His-Arg-Met-Ile-lys-leu-Thr-Thr-
Gly-Lys-Glu-Ala-Ala-Leu-Ser-Tyr-Gly-Phe-Tyr-Gly-Cys-
X-Cys-Gly-Val-Gly-Gly-Arg-Gly-.
```

The comparison with the result in Example 3 reveals that the amino acids of 1st through 5th, 8th, 9th, 11th through 19th and 21st through 25th from the N-terminal coincide with each other. By the way, X in the above formula means an unidentified amino acid residue.

Possible application in industry

The present invention relates to protein having phospholipase $A_2$ activity.

The protein according to the present invention will be used as an antigen in the immunoassay for diagnosis of rheumatoid arthritis or for cancer therapy by causing inflammation on the cancer site utilizing its proinflammatory effect.

**Claims**

1. Proteins with phospholipase $A_2$ activity having the amino acid sequence at the N-terminal of formula [I]:

```
NH2-Asn-Leu-Val-Asn-Phe-X-X-Met-Ile-X-Leu-Thr-Thr-Gly-Lys-
Glu-Ala-Ala-Leu-X-Tyr-Gly-Phe-Tyr-Gly-                    [I].
```

2. Proteins with phospholipase $A_2$ activity according to Claim 1 wherein the proteins originate from human inflammatory sites and have a molecular weight of about 13,700.

3. Polypeptides having the amino acid sequences containing at least the sequence of formula [I]:

```
NH2-Asn-Leu-Val-Asn-Phe-X-X-Met-Ile-X-Leu-Thr-Thr-Gly-Lys-
Glu-Ala-Ala-Leu-X-Tyr-Gly-Phe-Tyr-Gly-                    [I].
```

4. Polypeptides and their fragments having the amino acid sequence of formula [I]:

```
NH2-Asn-Leu-Val-Asn-Phe-X-X-Met-Ile-X-Leu-Thr-Thr-Gly-Lys-
Glu-Ala-Ala-leu-X-Tyr-Gly-Phe-Tyr-Gly-                    [I].
```

5. Proteins with phospholipase $A_2$ activity having amino acid sequence at the N-terminal of formula [II]:

```
NH2-Asn-Leu-Val-Asn-Phe-His-Arg-Met-Ile-Lys-Leu-Thr-Thr-
Gly-Lys-Glu-Ala-Ala-Leu-Ser-Tyr-Gly-Phe-Tyr-Gly-Cys-X-
Cys-Gly-Val-Gly-Gly-Arg-Gly                               [II].
```

6. Proteins with phospholipase $A_2$ activity according to Claim 5 wherein the proteins originate from human inflammatory sites and have a molecular weight of about 13,700.

7. Polypeptides having the amino acid sequences containing at least the sequence of formula [II]:

```
NH2-Asn-Leu-Val-Asn-Phe-His-Arg-Met-Ile-Lys-Leu-Thr-Thr-Gly-
Lys-Glu-Ala-Ala-Leu-Ser-Tyr-Gly-Phe-Tyr-Gly-Cys-X-Cys-Gly-
Val-Gly-Gly-Arg-Gly-                                   [II].
```

8. Polypeptides and their fragments having the amino acid sequence of formula [II]:

```
NH2-Asn-Leu-Val-Asn-Phe-His-Arg-Met-Ile-Lys-Leu-Thr-Thr-Gly-
Lys-Glu-Ala-Ala-Leu-Ser-Tyr-Gly-Phe-Tyr-Gly-Cys-X-Cys-Gly-
Val-Gly-Gly-Arg-Gly-                                   [II].
```

Fig. 1

Fig. 2

Fig. 4

Fig. 3

Fig. 5

Fig. 6

EP 0 446 350 A1

EP 0 446 350 A1

Fig. 7

$\frac{1}{V}$
$(\times 10^4 nmol^{-1} min \cdot mg)$

PC

PE

$1/S(1/mM)$

Fig. 8

Phospholipase $A_2$ Activity

(nmol /min/ml)

sever    slight    Osteoarthritis

Rheumatoid Arthritis

# INTERNATIONAL SEARCH REPORT

International Application No   PCT/JP88/01311

## I. CLASSIFICATION OF SUBJECT MATTER (if several classification symbols apply, indicate all) [6]

According to International Patent Classification (IPC) or to both National Classification and IPC

$$Int.Cl^4 \qquad C12N9/16$$

## II. FIELDS SEARCHED

### Minimum Documentation Searched [7]

| Classification System | Classification Symbols |
|---|---|
| IPC | C12N9/16 |

### Documentation Searched other than Minimum Documentation to the Extent that such Documents are Included in the Fields Searched [8]

Biological Abstracts Data Base (BIOSIS)
Protein Sequence Data Base (NBRF-PDB)

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | J. Pheumatol., Vol. 12, No. 2, (1985) Waldemar Pruzanski et al. [Phospholipase A2 Activity in Sera and Synovial Fluids in Rheumatoid Arthritis and Osteoarthritis. Its Possible Role as a Proinflammatory Enzyme] P.211-216 | 1-8 |
| A | J. Biochem., Vol. 100, (1986) Eva Stefanski et al. [Purification of a Soluble Phospholipase A2 from Synovial Fluid in Rheumatoid Arthritis] P.1297-1303 | 1-8 |
| P | J. Biochem., Vol. 104, (1988) Shuntaro Hara et al. [Amino Acid Composition and NH2-Terminal Amino Acid Sequence of Human Phospholipase A2 Purified from Pheumatoid Synovial Fluid] P.326-328 | 1-8 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| March 17, 1989 (17. 03. 89) | March 27, 1989 (27. 03. 89) |
| International Searching Authority | Signature of Authorized Officer |
| Japanese Patent Office | |

Form PCT/ISA/210 (second sheet) (January 1985)